(19) Europäisches Patentamt
European Patent Office
Office européen des brevets

(11) Publication number: **0 266 896 B1**

(12) # EUROPEAN PATENT SPECIFICATION

(45) Date of publication of patent specification: **05.08.92**

(51) Int. Cl.⁵: **C07D 471/04**, A61K 31/435, //(C07D471/04,221:00,205:00)

(21) Application number: **87308713.4**

(22) Date of filing: **01.10.87**

(54) **7-( (Meta-substituted) phenylglycine) 1-carba-1-dethiacephalosporins.**

(30) Priority: **03.10.86 US 915173**

(43) Date of publication of application:
**11.05.88 Bulletin 88/19**

(45) Publication of the grant of the patent:
**05.08.92 Bulletin 92/32**

(84) Designated Contracting States:
**AT BE CH DE ES FR GB GR IT LI NL SE**

(56) References cited:
**EP-A- 0 014 475**
**EP-A- 0 014 476**
**EP-A- 0 154 253**
**EP-A- 0 211 540**
**US-A- 3 697 515**

(73) Proprietor: **ELI LILLY AND COMPANY**
**Lilly Corporate Center**
**Indianapolis Indiana 46285(US)**

(72) Inventor: **Cooper, Robin David Grey**
**6740 Dover Road**
**Indianapolis, Indiana 46220(US)**
Inventor: **Webber, John Alan**
**7635 Cape Cod Circle**
**Indianapolis, Indiana 46250(US)**

(74) Representative: **Hudson, Christopher Mark et al**
**Erl Wood Manor**
**Windlesham Surrey GU20 6PH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel β-lactam antibiotics having improved therapeutic properties.

In U.S. Patent Specification No. 4,335,211 there is described a class of β-lactam antibiotics having a methylene group at the position normally occupied by a sulphur radical in classical cephalosporin molecules. Despite the considerable synthetic difficulties which exist, in view of their pharmacological properties, there is presently intense interest in such "carbacephalosporins". The main focus of the aforementioned U.S. Patent is concerned with an enzymatic method of producing optically pure unsubstituted and para-hydroxy-phenylglycyl derivatives. However, in passing, there is also mentioned the possibility of substituting the phenylglycyl group with a methanesulphonamido group.

Surprisingly, it has now been discovered that the substitution of the phenylglycyl moiety at the meta-position in this type of compound results in dramatically improved pharmacokinetic properties.

Thus, according to the present invention there is provided a compound of the Formula I:

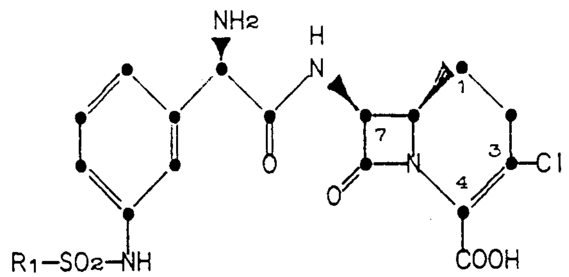

I

wherein $R_1$ is $C_1$ to $C_4$ alkyl, or a pharmaceutically-acceptable salt thereof.

Examples of the term "$C_1$ to $C_4$ alkyl" include methyl, ethyl, n-propyl, i-propyl, n-butyl, iso-butyl, and t-butyl. However, the simpler members of this group, in other words, the methyl and ethyl analogs, show particular promise. Thus, the methyl and ethyl analogs (i.e., $R_1$ is methyl and ethyl) exhibit a unique combination of excellent antibiotic activity and highly advantageous pharmacokinetic properties. These superior properties will be discussed in conjunction with Tables 1 and 2 which appear later in the specification.

The two preferred compounds of the invention are:

7β-[2′-(R)-2′-(m-(Methylsulfonamido)phenyl)-2′-aminoacetamido]-3-chloro-3-(1-carba-1-dethiacephem)-4-carboxylic acid; and

7β-[2′-(R)-2′-(m-(Ethylsulfonamido)phenyl)-2′-aminoacetamido]-3-chloro-3-(1-carba-1-dethiacephem)-4-carboxylic acid.

The term "pharmaceutically-acceptable salt" refers to salts formed from standard acid-base reactions with the carboxy group at the 4 position and/or the amino group at the 2′ position of the compounds of Formula I. Carboxylate salts can be formed between the carboxylate anion at the 4 position and a positive counter-ion. The counter-ions are preferably chosen from the alkali and alkaline earth metals, (such as lithium, sodium, potassium, barium and calcium); ammonium; and the organic cations (such as dibenzylammonium, benzylammonium, 2-hydroxyethylammonium, bis(2-hydroxyethyl)ammonium, phenylethylbenzylammonium, dibenzylethylenediammonium, and like cations). Other cations encompassed by the above term include the protonated form of procaine, quinine and N-methylglucosamine, and the protonated forms of basic amino acids such as glycine, ornithine, histidine, phenylglycine, lysine, and arginine.

The term "salt" also includes salts that form by standard acid-base reactions with basic groups (such as the (2') amino group) and organic or inorganic acids. Such acids include hydrochloric, sulfuric, phosphoric, acetic, trifluoroacetic, succinic, citric, lactic, maleic, fumaric, palmitic, cholic, pamoic, mucic, D-glutamic, d-camphoric, glutaric, phthalic, tartaric, lauric, stearic, salicyclic, methanesulfonic, benzenesulfonic, sorbic, picric, benzoic, cinnamic, and like acids.

Those skilled in the art will immediately appreciate that the compounds of the invention can exist in the form of internal salts ("zwitterions") in view of the presence of the 2'-amino group and the 4-carboxylic acid group. Such zwitterions of the compounds of Formula I form part of this invention.

The compounds of Formula I may also exist as solvates and hydrates. Thus, these compounds may crystallize with, for example, waters of hydration, or one, a number of, or any fraction thereof of molecules of the mother liquor solvent. The solvates and hydrates of such compounds are included within the scope of this invention.

In a further aspect of this invention there is provided a pharmaceutical composition comprising a compound of Formula I, or a pharmaceutically-acceptable salt thereof, associated with a pharmaceutically-acceptable carrier therefor.

With regard to compositions for oral administration (such as tablets and capsules), the term "pharmaceutically-acceptable carrier" includes common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils and colloidal silica. Flavoring agents such as peppermint, oil of wintergreen, cherry flavoring or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "pharmaceutically-acceptable carrier" includes conventional additives such as suspending agents, for example, sorbitol, syrup, methyl cellulose, glucose/sugar syrup, gelatin, hydroxyethyl-cellulose, carboxymethyl cellulose, aluminum stearate gel or hydrogenated edible oils, for example almond oil, fractionated coconut oil, oily esters, propylene glycol or ethyl alcohol; and preservatives such as methyl or propyl p-hydroxybenzoates or sorbic acid.

The preferred pharmaceutical compositions according to the present invention are those adapted for oral administration.

The pharmaceutical composition can also be for intravenous (IV) use. Specifically, a water soluble form of the antibiotic compound can be dissolved in one of the commonly used intravenous fluids and administered by infusion. When used in conjunction with compositions for IV use, the term "pharmaceutically-acceptable carrier" includes such fluids as physiological saline, Ringer's solution or 5% dextrose solution.

For intramuscular preparations a sterile formulation of a suitable salt form of the antibiotic compound (for example, the hydrochloride salt or sodium salt) can be formulated with a "pharmaceutically-accept able carrier". Examples of such sterile formulations are a suitable salt form either dissolved in a pharmaceutical diluent (for example, Water-for-Injection, physiological saline, 5% glucose) or suspended in an aqueous base or a pharmaceutically acceptable oil base (for example, an ester of a long chain fatty acid such as ethyl oleate).

Topical compositions can be formulated with a "pharmaceutically-acceptable carrier" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with a "pharmaceutically-acceptable carrier" such as a long- or quick-release base.

The compounds of Formula I can also be formulated in unit dosage form in sterile vials, sterile plastic pouches containing a port with a septum, or sterile, hermetically sealed ampoules. The compound of Formula I (or the corresponding pharmaceutically-acceptable salt) may be a dry powder or in crystalline or lyophylized form. The amount of the antibiotic compound per unit dosage may vary from about 100 milligrams to about 10 grams.

A therapeutically effective amount of the compounds of Formula I is generally from approximately 2.5 mg to about 50 mg of compound per kilogram of body weight per dose. This amount generally totals from about 0.25 gram to about 12 grams per day for an adult human.

The compounds of Formula I are useful for treating or controlling infectious diseases caused by gram-positive and gram-negative bacterial organisms in warm-blooded animals. This method comprises administering to the infected host a therapeutically effective amount of the antibiotic compound.

In practicing this method, the antibiotic compound can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance to the compounds of Formula I of both the patient and the microorganism or microorganisms involved in the infection.

The antibiotic activity of selected compounds of Formula 1 and, for comparison, a compound of related structure, is illustrated by the following in vitro test data. In Table I, the minimum inhibitory concentrations (MIC) for the compounds against a wide range of gram-positive and gram-negative bacteria are presented. The MIC values were obtained by the standard agar dilution test method. The excellent in vitro activity of the claimed compounds is especially surprising in light of their high solution stability. High solution stability of a β-lactam compound was until now thought to indicate low acylating ability of the β-lactam, and thus low antibacterial activity for the compound.

The antibiotic activity of the ortho analog of the methyl compound of the invention is presented in Table 1 as compound 3. Note, in particular, the dramatic difference the position of the $R_1$ substituent has on the antibiotic activity of this class of compounds.

**Table 1**

**Comparative In Vitro Antibiotic Activity of Select Compounds vs. Gram-Positive and Gram-Negative Bacteria**

| Test Organism* | Test Compound[5] Minimum Inhibitory Concentration (mcg/ml) 1 | 2 | 3 |
|---|---|---|---|
| Staphylococcus aureus X1.1 | 1 | 2 | 64 |
| Staphylococcus aureus V41[4] | 4 | 8 | 128 |
| Staphylococcus aureus S13E[1,4] | 2 | 2 | 64 |
| Staphylococcus epidermidis EPI1[3] | 4 | 8 | 128 |
| Staphylococcus epidermidis EPI2[3] | 2 | 4 | 128 |
| Streptococcus pyogenes C203 | 0.25 | 0.25 | 16 |
| Streptococcus pneumoniae Park I | 1 | 2 | 64 |
| Hemophilus influenzae C.L. | 2 | 2 | 16 |
| Escherichia coli N10 | 0.5 | 2 | 128 |
| Escherichia coli EC14 | 0.25 | 0.25 | 64 |
| Escherichia coli TEM[1] | 0.25 | 0.25 | 64 |
| Klebsiella pneumoniae X26 | 0.25 | 0.25 | 32 |
| Klebsiella pneumoniae X68 | 0.25 | 0.5 | 64 |
| Enterobacter aerogenes C32 | 2 | 4 | 128+ |
| Enterobacter aerogenes B17 | 1 | 2 | 128+ |
| Enterobacter cloacae EB5 | 4 | 8 | 128+ |

**Table 1 cont'd.**

Comparative *In Vitro* Antibiotic Activity of Select Compounds vs. Gram-Positive and Gram-Negative Bacteria

| Test Organism* | Test Compound[5] Minimum Inhibitory Concentration (mcg/ml) | | |
|---|---|---|---|
| | 1 | 2 | 3 |
| Salmonella typhi X514 | 0.5 | 2 | 128 |
| Salmonella typhi 1335 | 0.5 | 2 | 128 |
| Shigella sonnei N9 | 0.5 | 2 | 128 |

*Numerals and letters following the names of test microorganisms refer to the strains.

[1] β-lactamase producer

[3] methicillin resistant

[4] penicillin G resistant

[5] Test compounds numbered 1, 2 and 3 are as follows:

1. 7-(R)-[2'-(R)-2'-amino-2'-(m-(methylsulfonamido)phenyl)acetamido]-3-chloro-3-(1-carba-1-dethiacephem)-4-carboxylic acid, zwitterionic salt.

2. 7-(R)-[2'-(R)-2'-amino-2'-(m-(ethylsulfonamido)phenyl)acetamido]-3-chloro-3-(1-carba-1-dethiacephem)-4-carboxylic acid, zwitterionic salt.

3. 7-(R)-[2'-(R)-2'-amino-2'-(o-(methylsulfonamido)phenyl)acetamido]-3-chloro-3-(1-carba-1-dethiacephem)-4-carboxylic acid, zwitterionic salt.

A further characteristic of the compounds of this invention are the superior in vivo blood levels exhibited in test animals such as rats. For example, the superior in vivo pharmacokinetics of the compounds of the invention are set forth in the following Table II. In this Table, the intravenous pharmacokinetics in the rat of the methyl and ethyl compounds of Formula I, along with three other comparator compounds, are set forth. (The three comparator compounds are the paraisomer of the methyl compound of Formula I, and the unsubstituted phenylglycyl analog and the p-hydroxyphenylglycyl analog of the compounds of Formula I. The unsubstituted phenylglycyl analog and the p-hydroxyphenylglycyl analog are exemplified in U.S. Patent No. 4,335,211). The tests were performed by methods well-recognized in the art. (See, for example, Frank C. Tinsley et al., J. Appl. Physiol.: Respirat. Environ. Exercise Physiol. (54/5): 1422-1426 (1983).

Specifically, the male Sprague-Dawley rats were administered a 20 mg/kg dose intravenously. The plasma levels of the administered compound were monitored by bioassay with an E. coli strain. The bioassay results are presented below as obtained from a model-independent analysis:

Table II

Plasma Parameters in Rats on IV Dosing

| Test Compound[a] | Animals/-Time Point | AUC[b] | Half-Life (minutes) |
|---|---|---|---|
| 1 | 5 | 285 | 334 |
| 2 | 6 | 119 | 317 |
| 3 | 5 | 18 | 33 |
| 4 | 6 | 12 | 41 |
| 5 | 6 | 20 | 39 |

[a] Test compounds numbered 1, 2, 3, 4 and 5 are as follows:

1. 7-(R)-[2'-(R)-2'-amino-2'-(m-(ethylsulfonamido)-phenyl)acetamido]-3-chloro-3-(1-carba-1-dethiacephem)-4-carboxylic acid, zwitterionic salt.
2. 7-(R)-[2'-(R)-2'-amino-2'-(m-(methylsulfonamido)-phenyl)acetamido]-3-chloro-3-(1-carba-1-dethiacephem)-4-carboxylic acid, zwitterionic salt.
3. 7-(R)-[2'-(R)-2'-amino-2'-(p-(methylsulfonamido)-phenyl)acetamido]-3-chloro-3-(1-carba-1-dethiacephem)-4-carboxylic acid, zwitterionic salt.
4. 7-(R)-[2'-(R)-2'-amino-2'-phenylacetamido]-3-chloro-3-(1-carba-1-dethiacephem)-4-carboxylic acid, zwitterionic salt.
5. 7-(R)-[2'-(R)-2'-amino-2'-(p-hydroxyphenyl)-acetamido]-3-chloro-3-(1-carba-1-dethiacephem)-4-carboxylic acid, zwitterionic salt.

[b] AUC = area under the curve

The excellent activity and pharmacokinetic profile of the compounds of this invention render them useful for the therapeutic or prophylactic treatment of infections in warm-blooded animals caused by gram-positive, gram-negative, and acid-fast bacteria.

The antibiotic compounds can be administered orally, parenterally (e.g. intravenously, intramuscularly or subcutaneously) or as a topical ointment or solution. Oral administration is preferred.

In a further aspect of the invention there is provided a process for preparing a compound of formula I, or a pharmaceutically-acceptable salt thereof, which comprises:

(A) the removal of the amino and/or carboxy protecting group from a compound of the formula II:

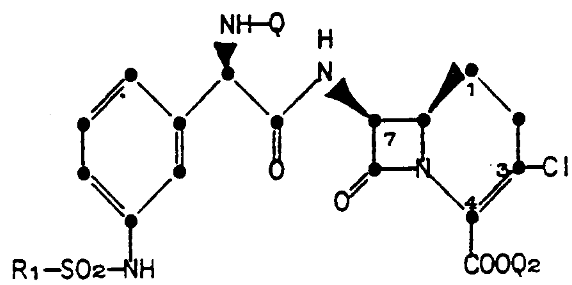

II

wherein Q is hydrogen or an amino-protecting group, and $Q_2$ is hydrogen or a carboxy-protecting group, provided that both Q and $Q_2$ are not hydrogen; or

(B) acylation of a compound of the formula III

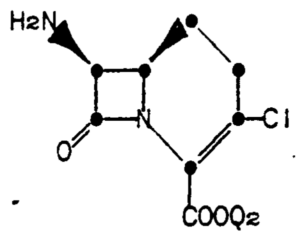

III

wherein $Q_2$ is as defined above, followed, in the case where $Q_2$ is not hydrogen, by removal of the carboxy protecting group and any amino-protecting group present;

and

(C) resolution of a racemic compound of formula II where Q and $Q_2$ are hydrogen.

The carboxy-protecting group $Q_2$ of formula II is a conventional carboxy-protecting group and preferably one which is not sterically hindered. Examples of such groups are benzyl and substituted benzyl groups such as 4-methoxybenzyl, 4-nitrobenzyl, 4-methylbenzyl, 3,5-dimethylbenzyl, and 4-chlorobenzyl; silyl group such as a trialkylsilyl group (trimethylsilyl); and halo-substituted alkyl groups such as the 2,2,2-trichloroethyl, 2,2,2-tribromoethyl, and 2-iodoethyl groups. A preferred ester group is the benzyl or a substituted benzyl ester group.

The methods for the acylation of the 7β-amino nucleus compounds of formula III with an acyl side chain are similar to the methods for the acylation of 6-amino-penicillanic acid, 7-aminodesacetoxycephalosporanic acid, and 7-aminocephalosporanic acid.

In general, the acylation can be effected by reacting an acylating agent of the formula

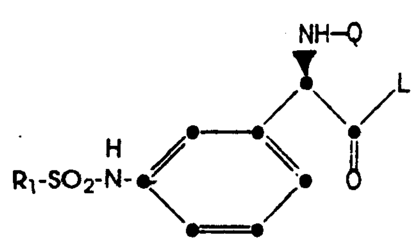

wherein $R_1$ is $C_1$ to $C_4$ alkyl, Q is hydrogen or, preferably, an amino-protecting group and L is a good leaving group (such as chloro, bromo, or an activated ester). The unprotected acylating agent can also be used in the form of an acid-addition salt, for example, the hydrochloride. The group L can be formed in situ from the free acid. The acylation is typically carried out in an aprotic organic solvent from about 15 ° C to about 50 ° C. Suitable solvents include methylene chloride.

One method is to simply combine the 7β-amino nucleus with an acid chloride or acid bromide in the presence of an acid scavenger. The acid chloride or acid bromide may be formed in situ. Another method

is to combine the 7$\beta$-amino nucleus with the free carboxylic acid form of the side chain (or its acid salt) and a condensing agent. Suitable condensing agents include N,N'-disubstituted carbodiimides such as N,N'-dicyclohexylcarbodiimide, N,N'-diethylcarbodiimide, N,N'-di(n-propyl)carbodiimide, N,N'-di(iso-propyl)-carbodiimide, N,N'-diallylcarbodiimide, N,N'-bis(p-dimethylaminophenyl)-carbodiimide, N-ethyl-N'-(4"-ethylmorpholinyl)carbodiimide, and the like. Other suitable carbodiimide condensing agents are disclosed by Sheehan in U.S. Patent No. 2,938,892 and by Hofmann et al. in U.S. Patent No. 3,065,224. Azolides, such as N,N'-carbonyldiimidazole and N,N'-thionyldiimidazol, may also be used as condensing agents. Dehydrating agents such as phosphorus oxychloride, the alkoxyacetylenes, and 2-halogenopyridinium salts (such as 2-chloropyridinium methyl iodide, 2-fluoropyridinium methyl iodide, and the like) may be used to couple the free acid or its acid salt with the 7$\beta$-amino nucleus.

Another acylation method entails first converting the free carboxylic acid form (or the corresponding salt) of the acyl side chain to the corresponding active ester derivative, which is in turn used to acylate the nucleus. The active ester derivative is formed by esterifying the free acid form with groups such as p-nitrophenol, 2,4-dinitrophenol, trichlorophenol, pentachlorophenol, 2-chloro-4,6-dimethoxytriazene, N-chlorosuccinimide, N-chloro maleic imide, N-chlorophthalimide, 1-hydroxy-1H-benzotriazole or 1-hydroxy-6-chloro-1H-benzotriazole. The active ester derivatives can also be mixed anhydrides, which are formed with groups such as methoxycarbonyl, ethoxycarbonyl, isobutoxycarbonyl, trichloromethylcarbonyl, and iso-but-2-ylcarbonyl, and the carboxylic acid of the acyl side chain. The mixed anhydrides are synthesized by acylating the carboxylic acid of the acyl side chain.

Alternatively, the 7$\beta$-amino nucleus can be acylated with the N-ethoxycarbonyl-2-ethoxy-1,2-dihydroquinoline (EEDQ) derivative of the acyl side chain. In general, the free acid form of the acyl side chain and EEDQ are reacted in an inert, polar organic solvent (such as tetrahydrofuran, acetonitrile, and the like). The resultant EEDQ derivative is used in situ to acylate the 7$\beta$-amino nucleus.

Yet another method of acylating the 7$\beta$-amino compounds entails the use of an enzymatically-assisted process. Such a process is described in U.S. Patent No. 4,335,211.

A preferred method of acylation involves suspending the hydrochloride salt of a compound of Formula III in methylene chloride. N-methylmorpholine and the amino-protected side chain (as the free acid) are added and the solution is cooled. Pyridine and phosphoryl chloride is added to generate in situ the acid chloride form of the side chain. The reaction is stirred at room temperature until it is substantially complete and the acylated nucleus product is isolated in a conventional manner.

The amino- and carboxy-protecting groups can be removed by methods well known in the art. Examples of conditions for the removal of these two types of protecting groups can be found in standard words such as E. Haslam, "Protective Groups in Organic Chemistry", J.G.W. McOmie, Ed., Plenum Press, New York, N.Y., 1973, Chapters 2 and 5, and T.W. Greene, "Protective Groups in Organic Synthesis", John Wiley and Sons, New York, N.Y., 1981, Chapters 5 and 7, respectively.

Examples of procedures for the removal of amino- and carboxy-protecting groups can also be found in the Experimental Section. For example, the t-butoxy carbonyl amino-protecting group was removed with trifluoroacetic acid, and the p-nitrobenzyl carboxy-protecting group was removed by hydrogenolysis.

The resolution of a racemic mixture of a compound of formula II can also be effected by conventional means.

The compounds of formula II are novel and are provided in a further aspect of the invention.

The 7$\beta$-amino 3-chloro 1-carba-1-dethiacephem compound of formula III can be synthesized from the corresponding 3-hydroxy compounds in accord with the process diagrammed below in Scheme 1:

Scheme 1

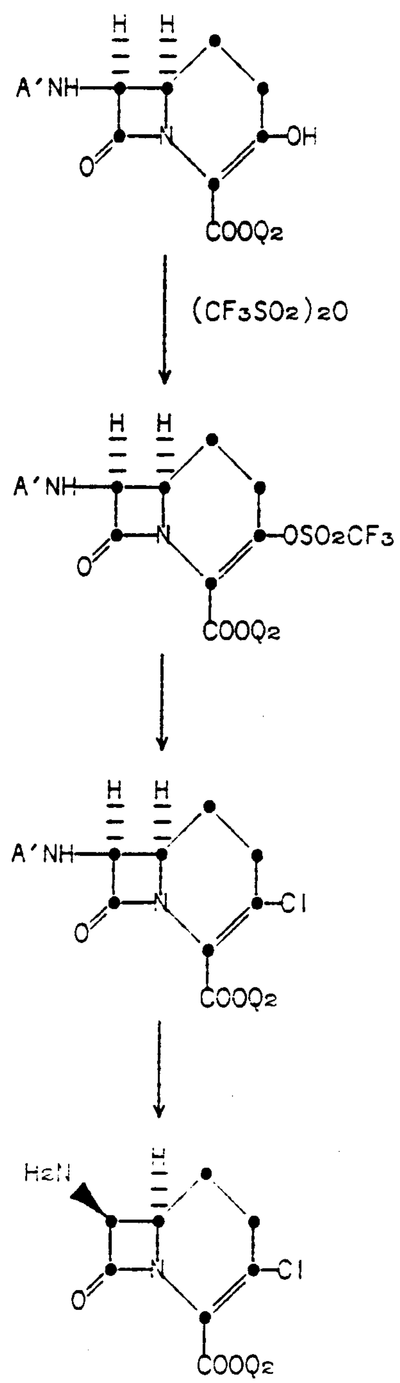


IIIb

IIIa

IIA

III

In the above Scheme 1, $Q_2$ is a carboxy-protecting and A′ is either an amino-protecting group or an acyl group.

The sulfonylation of the 3-hydroxy group represented by the first reaction in above Scheme I (Formula IIIb→Formula IIIa) can be carried out in an inert solvent at a temperature between about 0 ° C and about 35 ° C in the presence of a tertiary amine. Amines which are suitable include triethylamine, tri-(n-butyl)-amine, pyridine, t-butyldiethylamine, di(isopropyl)ethylamine, and like amines. Hindered trialkylamines are preferred. The acylating reagent can be trifluoromethanesulfonic anhydride (triflic anhydride), trifluoromethanesulfonyl chloride (triflic chloride) or other suitable acid derivative of trifluoromethanesulfonic acid. Inert solvents useful in the process are the halogenated hydrocarbons such as chloroform, methylene

chloride, trichloroethane, and the like; ether solvents such as tetrahydrofuran; esters such as ethyl acetate; or other inert solvents such as acetonitrile (see, for example, European Patent No. 211,540).

The triflic esters (Formula IIIa) can be recovered from the reaction mixture by conventional isolation methods, such as by extraction.

During the acylation any reactive groups in the side chain group A′ also capable of acylation is desirably protected. For example, any amino group substituents are protected with a conventional amino-protecting group to prevent amide formation in competition with the desired sulfonylation formation.

The second, chlorination reaction in the above Scheme 1 (Formula IIIa→Formula IIA) can proceed with lithium chloride in an aprotic solvent at a temperature between about 60° C and about 95° C.

Aprotic polar solvents which can be used in the chlorination reaction are dimethylformamide, dimethylacetamide, N-methylpyrrolidone, acetonitrile, and like solvents. Dimethylformamide is a preferred solvent.

Preferably the reaction is carried out at a temperature between about 75° C and about 85° C with an excess of the stoichiometric amount of the lithium chloride salt.

A preferred carboxy-protecting group at $Q_2$ is the benzyl or a substituted benzyl group.

Following completion of the chlorination reaction the 3-halo-1-carba-3-cephem ester is recovered from the reaction mixture by conventional isolation methods and is purified by chromatography.

As with the first reaction in Scheme 1, during the chlorination reaction it is desirable to protect any amino groups present in the starting material. In an example of the reaction, benzyl 7β-phenoxyacetylamino-3-trifluoromethylsulfonyloxy-1-carba-1-dethiaceph m-4-carboxylate is dissolved in dimethylformamide and an excess (such as 3-4 molar excess) of lithium chloride is added. The solution is stirred and heated to a temerature of about 80° C for about 5-6 hours. The progress of the chlorination can be followed by thin layer chromatography of a small aliquot periodically removed from the reaction mixture. When the reaction is completed the mixture is diluted with a water-immiscible organic solvent, washed with water, dried, and evaporated. The crude product (benzyl 7β-phenoxyacetylamino-3-chloro-1-carba-1-dethiacephem-4-carboxylate) is purified by chromatography (for example, over silica gel).

The final reaction in the above Scheme 1, which is either the removal of the amino-protecting group or the cleavage of the amido group represented by the partial formula "A′NH-" (compound IIA→Compound III) are reactions well known in the art. The removal of amino-protecting groups are taught in the references mentioned above for the acylation and subsequent deprotection of the compounds of Formula III.

The methods for cleaving the amide bond of a 7-(amido) side chain are well known in the art. One such method employs nitrosyl chloride, as exemplified in U.S. Patent No. 3,507,862. Another method uses phosphorus pentachloride in the presence of a (preferably nitrogen) base. The latter process is described in, for example, U.S. Patent No. 3,549,628, U.S. Patent No. 3,697,515, and U.S. Patent No. 3,868,368.

An improved version of the above phosphorous pentachloride method can employ a triphenylphosphitechlorine kinetic reagent, as discussed in U.S. Patent No. 4,211,702. An excess (2 to 3 equivalents) of this reagent can be used to both cleave the 7-amido group and chlorinate the 3-hydroxy group of the 3-enol 1-carba-1-dethiacephem (for example, the compounds of Formula IIIb above) analogous to the conditions set forth in U.S. Patent No. 4,226,986.

Yet another method for synthesizing the 7-amino compounds of Formula III is the method set forth in U.K. Patent Specification No. 2,041,923A and its equivalents (such as European Patent Specification No. 14,475A). This method entails, in general, addition of a phenylthiol to a 7-azido 3-hydro-1-carba-1-dethia-4-carboxylate compound. The resulting 3-thio-3,4-saturated compound is oxidized to the corresponding 3-sulfoxide compound. The 3-sulfoxide compound is chlorinated and the resultant 3-sulfoxide-3-chloro compound is treated with base (to eliminate the sulfoxide) to give the 3-chloro-3-cephem compound.

The 3-hydroxy starting materials (Formula IIIb) of Scheme 1 can be prepared as described in U.S. Patent Specification No. 4,665,171 or European Patent Specification No. 209,352. (For a related discussion, see also D. A. Evans et al., Tetrahedron Letters, 26, pp. 3783-3786 and 3787-3790 (1985)).

This process is further described in European Patent No. 211,540, and in D. A. Evans et al., Tetrahedron Letters, 26, pp. 3787-3790 (1985).

A second method for making a 3-hydroxy 1-carba-1-dethiacephalosporin is set forth in M. Hatanaka et al., Tet. Letters, 24, pp. 4837-4838 (1983). The method of Hatanaka et al. is similar to that of Evans in that the β-lactam ring is first formed from a 2+2 cycloaddition. However, Hatanaka et al. form the 6-membered ring from an acetate ester fragment appended to the 1-position nitrogen which serves as a nucleophile in the Dieckmann condensation step.

In the following Preparation and Examples, the terms nuclear magnetic resonance spectra, high performance liquid chromatography, field desorption mass spectroscopy, fast atom bombardment mass spectroscopy, and specific rotation are abbreviated n.m.r., HPLC, f.d.m.s., f.a.b.m.s. and o.r., respectively.

In conjunction with the n.m.r. spectra, the following abbreviations are used: "s" is singlet, "d" is doublet, "dd" is doublet of doublets, "t" is triplet, "q" is quartet, and "m" is multiplet, respectively. "DMSO-$d_6$" is dimethyl sulfoxide where all protons have been replaced with deuterium.

The n.m.r. spectra were obtained on a Varian Associates EM-390 90 MHz instrument, on a Jeol FX-90Q 90 MHz instrument, on a Bruker Corporation 270 MHz or 500 MHz instrument, or on a General Electric QE-300 300 MHz instrument. The chemical shifts are expressed in δ values (parts per million downfield from tetramethylsilane).

Preparation 1

m-(Methylsulfonamido)Benzaldehyde

m-(Amino)benzaldehyde polymer (1210 g, 10.0 mole), THF (12,000 ml), and water (495 ml) were combined and stirred overnight at room temperature. The resultant slurry was cooled to between about 5 to about 10° C and pyridine (1580 g, 20 moles) was added. Methylsulfonyl chloride (2290 g, 20 moles) was added in a dropwise fashion to the slurry while maintaining the temperature of reaction solution at approximately 10° C. The reaction was stirred in an ice bath overnight, during which time the ice in the bath melted and the temperature of the reaction climbed to between about 15° to 20° C. The THF was removed in vacuo and 1N hydrochlor ic acid (6 l) was added to the residue. The resultant slurry was stirred at room temperature for approximately 8 hours. The orange, granular material of the slurry was collected on a filter, was washed with water (approximately 6 liters) and was dried for 48 hours at 40° C to yield 1833 g of material. A portion of this material (940 g) was recrystallized from an ethyl acetate mixture containing carbon black and sodium carbonate. Two crops of crystals from this solution yielded 874 g of m-(methylsulfonamido)benzaldehyde: n.m.r. (360 MHz, DMSO-$d_6$): δ 3.05 (s, 3), 7.5-7.9 (m, 4), 9.9 (s, 1), 10.1 (s, 1).

Preparation 2

D,L-2-(Amino)-2-(m-(Methylsulfonamido)phenyl)acetonitrile

Concentrated ammonium hydroxide (43 ml) was cooled to 10° C. Sodium cyanide (5.4 g, 110 mmol), ammonium chloride (5.5 g, 102 mmol), and m-(methylsulfonamido)benzaldehyde (10.0 g, 50 mmol) were added and the resultant solution was stirred for 4 hours between about 10° to 15° C. Excess ammonia of the solution was removed in vacuo at 15° C. The pH of the residue was adjusted to 7.0 by the addition of concentrated hydrochloric acid. The resultant solution was extracted with ethyl acetate (6X) and the ethyl acetate extractions were combined, washed with brine (2X), dried over magnesium sulfate, filtered, and evaporated in vacuo to yield 10.6 g, 94 % of a brown oil of D,L-2-(amino)-2-((m-methylsulfonamido)phenyl)-acetonitrile: n.m.r. (DMSO-$d_6$, 90 MHz): δ 3.0 (s, 3), 5.0 (s, 1), 7.2-7.6 (m, 4).

Preparation 3

2-(R)-2-(Amino)-2-(m-(Methylsulfonamido)phenyl)acetonitrile, L-Tartaric Acid Salt, Acetic Acid Solvate

L-(+)-Tartaric acid (8.25 g, 55 mmol) was dissolved in acetic acid (90%) and the mixture was heated to affect solution. The solution was added to D,L-2-(amino)-2-((m-methylsulfonamido)phenyl)acetonitrile (10.6 g, 47 mmol) and the resultant solution was allowed to cool slowly. Ethyl acetate (25 ml) was slowly added and the resultant precipitate was stirred overnight at room temperature then was collected by filtration. The collected precipitate was washed with acetic acid and ethyl acetate then dried at 36° C in vacuo to yield 5 g, 49% of 2-(R)-2-(amino)-2-(m-(methylsulfonamido)phenyl)acetonitrile, L-tartaric acid salt, acetic acid solvate: n.m.r.: ($D_2O$/DCl, 90 MHz): δ 2.1 (s, 1), 3.2 (s, 3), 4.8 (s, 1), 5.8 (s, 1), 7.3-7.7 (m, 4); o.r.

$$[\alpha]_D^{25^\circ} = +27.66^\circ$$

[1N hydrochloric acid, C 1].

Preparation 4

2-(R)-2-Amino-2-(m-(Methylsulfonamido)phenyl)acetic Acid, Hydrochloride Salt

2-(R)-2-(Amino)-2-(m-(methylsulfonamido)phenyl)acetonitrile, L-tartaric acid salt, acetic acid solvate (18 g, 48 mmol) was heated to reflux temperature in 6N hydrochloric acid (200 ml) for approximately 6 hours. After removal of excess hydrochloric acid under reduced pressure, carbon black was added and the suspension was filtered through a filter cell. The pH of the filtrate was adjusted to 5.0 by the addition of 5N sodium hydroxide solution. The filtrate was used in situ in Preparation 5.

Preparation 5

2-(R)-(N-(t-Butoxycarbonylamino)-2-(m-(Methylsulfonamidophenyl))acetic Acid

The pH of the solution from Preparation 4 above was adjusted to 9 by the addition of 5N sodium hydroxide solution. THF (300 ml) then di(t-butyl dicarbonate) (15.7 g, 72 mmol) was added and the pH of the solution was readjusted to 9.0 by the addition of 5N sodium hydroxide solution. The solution was stirred at room temperature for 48 hours then the THF was removed in vacuo. The resultant concentrate was washed with diethyl ether (2X), and the pH of the water layer was adjusted to 2 by the addition of 6N hydrochloric acid. The acidified aqueous layers were extracted with ethyl acetate (4X). The ethyl acetate layers were combined, dried over magnesium sulfate, filtered, and evaporated in vacuo to give 11 g of foam. The foam was chromatographed by preparatory-scale HPLC on a silica column eluted with a gradient of toluene versus 50% ethyl acetate/toluene to give 6.85 g, 41% of an 84:16 R:S mixture of 2-(R)-(N-(t-butoxycarbonylamino))-2-(m-(methylsulfonamido)phenyl)acetic acid: n.m.r. (300 MHz, $CDCl_3$): 1.3 (s, 9), 5.1 (d, 1), 5.3 (d, 1), 7.1-7.5 (m, 4), 7.9 (d, 1); o.r.:

$$[\alpha]_D^{25^\circ} = -74.4^\circ$$

[methanol, C 1].

Preparation 6

p-Nitrobenzyl 7β-amino-3-Chloro-3-(1-Carba-1-dethiacephem)-4-Carboxylate Hydrochloride Salt

Under a nitrogen atmosphere, p-Nitrobenzyl 7β-[phenoxyacetamido]-3-chloro-3-(1-carba-1-dethiacephem)-4-carboxylate (770 mg, 1.58 mmol) (see Example 5, European Patent No. 211540), was dissolved in methylene chloride (15 ml). Pyridine (160 microliters, 1.98 mmol) then phosphorus pentachloride (380 mg, 1.82 mm) were added and the reaction solution was stirred for four hours at room temperature under nitrogen. iso-Butanol (1.1 ml, 11.85 mmol) was added and the solution was stirred until crystals formed. The mixture was cooled to between 0° and 5° C by an external ice bath and stirred for an additional hour. The crystals were collected by filtration, washed with cold methylene chloride, dried in vacuo at room temperature overnight to yield 568 mg, 93% yield of the title product.

Preparation 7

p-Nitrobenzyl 7β-[2'-(R)-2'-(m-(Methylsulfonamido)phenyl)-2'-(N-(t-Butoxycarbonylamino))acetamido]-3-Chloro-3-(1-Carba-1-Dethiacephem)-4-Carboxylate

p-Nitrobenzyl 7β-amino-3-chloro-3-(1-carba-1-dethiacephem)-4-carboxylate hydrochloride salt (194 mg, 0.5 mmol) was suspended in methylene chloride (3.5 ml) then N-methylmorpholine (55. 5 µl) was added. 2-(R)-2-(m-methylsulfonamido)phenyl)-2-[N-(t-butoxycarbonylamino)]acetic acid (172 mg, 0.5 mmol) was added and the solution was cooled to 0° C. Pyridine (170 µl, 2.1 mmol) then phosphoryl chloride (79.1 µl, 0.85 mmol) was added and the solution was stirred for 2 hours at approximately 0° to approximately 5° C. 1N Hydrochloric acid (10 ml) was added and the methylene chloride was removed under reduced pressure. Ethyl acetate (50 ml) was added and the solution was washed with 1N Hydrochloric acid (2X), brine, dried over magnesium sulfate, filtered, and evaporated in vacuo to yield 245 mg of a foam. The foam was chromatographed by HPLC on a silica gel column, eluted with a mixture of 48%/48%/4% ethyl acetate/hexane/isopropanol to yield 165 mg, 49% of p-nitrobenzyl 7β-[2'-(R)-2'-(m-(methylsulfonamido)-

phenyl)-2′-(N-(t-butoxycarbonylamino))acetamido]-3-chloro-3-(1-carba-1-dethiacephem)-4-carboxylate: n.m.r. (300 MHz, $CDCl_3$): δ 1.4 (s, 9), 1.6-1.9 (m, 2), 2.5-2.8 (m, 2), 3.0 (s, 3), 3.8-4.0 (m, 1), 5.2 (d, 1), 5.4 (q, 2), 5.9 (q, 1), 7.1-7.4 (m, 4), 7.6 (d, 2), 8.2 (d, 2).

Preparation 8

7β-[2-(R)-2′-(m-(Methylsulfonamido)phenyl)-2′-(N-(t-Butoxycarbonylamino))acetamido]-3-Chloro-3-(1-Carba-1-Dethiacephem)-4-Carboxylic acid

Five percent palladium on carbon (150 mg) was wetted with ethanol, then p-nitrobenzyl 7β-[2′-(R)-2′-(m-(methylsulfonamido)phenyl)-2-(N-(t-butoxycarbonylamino))acetamido]-3-chloro-3-(1-carba-1-dethiacephem)-4-carboxylate (300 mg, 0.44 mmol) (dissolved in a minimum amount of ethyl acetate) was added. The resultant suspension was hydrogenated for 20 minutes at a hydrogen pressure of 40 psi. The suspension was filtered, then washed with 1N hydrochloric acid (3X), brine (2X), dried over magnesium sulfate, filtered, and evaporated in vacuo to a solid. The solid was stirred in diethyl ether for 48 hours then collected by filtration and dried in vacuo. The solid was crystallized from diethyl ether, triturated with diethyl ether, filtered, then dried in vacuo to yield 50 mg, 62% of 7β-[2-(R)-2-(m-(methylsulfonamido)phenyl)-2-[N-(t-butoxycarbonylamino)acetamido]-3-chloro-3-[1-carba-1-dethiacephem]-4-carboxylic acid: f.d.m.s.: $M^+ + 1 = 543$.

Example 1

7β-[2′-(R)-2′-(m-(Methylsulfonamido)phenyl)-2′-Aminoacetamido]-3-Chloro-3-(1-Carba-1-Dethiacephem)-4-Carboxylic Acid Trifluoroacetate Salt

Trifluoroacetic acid (approximately 10 ml) was cooled to approximately 0°C. 7β-[2′-(R)-2′-(m-(methylsulfonamido)phenyl)-2′-[N-(t-butoxycarbonylamino)acetamido]-3-chloro-3-(1-carba-1-dethiacephem)-4-carboxylic acid (397 mg, 0.73 mmol) was added and the resultant solution was stirred at approximately 0°C for 20 minutes then warmed to room temperature. The solvent was evaporated in vacuo to give an oil. The oil was combined with diethyl ether and the resultant solid was triturated for 90 minutes with diethyl ether. The mixture was filtered and the solid dried in vacuo to yield 392 mg, 96% of 7β-[2′-(R)-2′-(m-(methylsulfonamido)phenyl)-2′-aminoacetamido]-3-chloro-3-(1-carba-1-dethiacephem)-4-carboxylic acid trifluoroacetate salt: n.m.r. (300 MHz, DMSO-$d_6$): δ 1.4 (m, 2), 2.3 - 2.7 (m, 2), 3.0 (s, 3), 3.8 (m, 1), 4.9 (s, 1), 5.4 (s, 1), 7.1-7.5 (m, 4), 3.3 (s, 2), 9.9 (broad, 1).

Example 2

7β-[2′-(R)-2′-(m-(Methylsulfonamido)phenyl)-2′-Aminoacetamido]-3-Chloro-3-(1-Carba-1-Dethiacephem)-4-Carboxylic Acid, Zwitterionic Salt

7β-[2′-(R)-2′-(m-(methylsulfonamido)phenyl)-2′-aminoacetamido]-3-chloro-3-(1-carba-1-dethiacephem)-4-carboxylic acid trifluoroacetate salt was chromatographed by preparatory-scale HPLC on a 20 μ $C_{18}$ reverse-phase silica column eluted with a gradient of 5% to 15% acetonitrile/1% acetic acid/water to yield 73.6 mg of 7β-[2′-(R)-2′-(m-(methylsulfonamido)phenyl)-2′-aminoacetamido]-3-chloro-3-(1-carba-1-dethiacephem)-4-carboxylic acid, zwitterionic salt: n.m.r. (270 MHz, DMSO-$d_6$) δ 1.48 (m, 2), 2.33 (m, 2), 2.99 (s, 3), 3.73 (m, 1, J = 4.7, 5.0, 10.0 Hz), 4.64 (s, 1), 5.26 (d, 1, J = 4.7 Hz), 7.1-7.4 (m, 4).

Preparation 9

m-(Ethylsulfonamido)benzaldehyde

m-(Amino)benzaldehyde (11.9 g, 0.1 mole), acetonitrile (50 ml), and water (1 ml) were combined, stirred vigorously, then cooled in an ice bath. Pyridine (16 ml, 0.2 mole) was added to the solution, followed by the dropwise addition of ethylsulfonyl chloride (25 g, 0.2 mole). The solution was stirred overnight at room temperature, then concentrated in vacuo. Five percent hydrochloric acid (200 ml) was added to the concentrate and the resultant solution was stirred for one hour. The solution was extracted with ethyl acetate (2X, 200 ml), the ethyl acetate extracts were combined and washed first with five percent hydrochloric acid solution then with brine. The extracts were dried over magnesium sulfate, filtered, and concentrated in

vacuo. The concentrate was treated with carbon tetrachloride. The resultant crystals were collected by filtration, then dried to yield 78%, 16.7 g of m-(ethylsulfonamido)benzaldehyde: n.m.r. (90 MHz, DMSO-$d_6$): δ 1.4 (t, 3), 3.2 (q, 2), 7.6 (m, 4), 10.0 (s, 1), 10.2 (s, 1).

## Preparation 10

D,L-2-(Amino)-2-(m-(Ethylsulfonamido)phenyl)acetic Acid, Hydrochloride Salt

Sodium cyanide (12 g, 0.24 mole), ammonium chloride (13.2 g, 0.24 mole), and ammonium hydroxide (20 ml, 24 g, 0.11 mole) were combined, stirred, and cooled. m-(Ethylsulfonamido)benzaldehyde (24 g) was added to the mixture and the resultant solution was stirred overnight at room temperature. The solution was poured into a mixture of ethyl acetate (200 ml) and water (600 ml) and the layers were separated. The aqueous layer was extracted with ethyl acetate (2X, 200 ml). The ethyl acetate extracts were combined, then washed with brine and 20% hydrochloric acid (4x, 50 ml). The acidic aqueous washes were combined, heated to reflux for 3 hours, concentrated hydrochloric acid (100 ml) was added and the resultant solution was heated to reflux for an additional 2 hours. The solution was cooled and then evaporated in vacuo to a slurry of D,L-2-(amino)-2-(m-(ethylsulfonamido)phenyl)acetic acid. The slurry was used without further purification in Preparation 11.

## Preparation 11

D,L-2-(N-(t-Butoxycarbonylamino))-2-(m-(Ethylsulfonamido)phenyl)acetic Acid

D,L-2-(Amino)-2-(m-(ethylsulfonamido)phenyl)acetic acid, hydrochloride salt (the slurry from Preparation 10) was dissolved in a 60:40 acetonitrile:water mixture (100 ml). The pH of the mixture was taken to 8.0 by the addition of 1N sodium hydroxide. Di(t-butyl)-dicarbonate (8.1 g, 1 equivalent) was added and the solution was stirred at room temperature for 72 hours. 1N Sodium hydroxide was added periodically to maintain the pH of the reaction solution at approximately 8.0. The reaction solution was concentrated in vacuo then saturated aqueous sodium bicarbonate solution (100 ml) was added. The mixture was extracted with ethyl acetate and in turn the ethyl acetate layer was washed with saturated aqueous sodium bicarbonate solution. The aqueous layers were combined and extracted once again with ethyl acetate. The combined aqueous layer was then layered with fresh ethyl acetate and the pH of the aqueous layer was adjusted to 2.5 by the addition of 20% hydrochloric acid. The mixture was then extracted with ethyl acetate (2X, 200 ml). The two ethyl acetate washings were combined and washed with brine, dried over magnesium sulfate, filtered, and concentrated in vacuo to give 5.7 g of crude product. The crude product was subjected to preparatory-scale HPLC on a silica gel column eluted with a gradient of tolune versus 50% ethyl acetate/toluene to yield 1.8 g of D,L-2-(N-(t-butoxycarbonamido))-2-(m-(ethylsulfonamido)phenyl)acetic acid: n.m.r. (90 MHz, DMSO-$d_6$): δ 1.18 (t, 3), 1.38 (s, 9), 3.08 (q, 2), 5.02 (d, 1), 7.18 (m, 4), 7.5 (d, 1), 9.8 (s, 1).

## Preparation 12

p-Nitrobenzyl 7β-[2′-(R,S)-2′-(m-(Ethylsulfonamido)phenyl)-2′-(N-(t-Butoxycarbonylamino))acetamido]-3-Chloro-3-(1-Carba-1-Dethiacephem)-4-Carboxylate

D,L-2-(t-Butoxycarbonylamino-2-(m-(ethylsulfonamido)phenyl))acetic acid (1.2 g, 0.003 mole) was dissolved in methylene chloride (20 ml). The solution was cooled in an ice bath then 2,4,6-chlorodimethoxytriazene (580 mg) and N-methylmorpholine (333 mg) as a methylene chloride solution (10 ml). The resultant solution was stirred in an ice bath for approximately 40 minutes.

p-Nitrobenzyl 7β-amino-3-chloro-(1-carba-1-dethiacephem)-4-carboxylate (1.2 g, 0.003 mole) was suspended in methylene chloride (15 ml) and the suspension was cooled in an ice bath. Triethylamine (0.42 ml, 1 equivalent) was added and the solution was stirred for approximately five minutes. This solution was added to the solution from the above paragraph and the resultant solution was stirred at room temperature for 45 minutes then concentrated in vacuo to an oil (1.78 g). The oil was evaporated in vacuo to a foam of p-nitrobenzyl 7β-[2′-(R,S)-2′-(m-(ethylsulfonamido)phenyl)-2′-(N-(t-butoxycarbonylamino))acetamido]-3-chloro-3-(1-carba-1-dethiacephem)-4-carboxylate: f.d.m.s.: $M^+ + 1 = 691$.

## Preparation 13

7β-[2′-(R,S)-2′-(m-(Ethylsulfonamido)phenyl)-2′-(N-(t-butoxycarbonylamino))acetamido]-3-Chloro-3-(1-Carba-1-Dethiacephem)-4-Carboxylic Acid

p-Nitrobenzyl 7β-[2′-(R,S)-2′-(m-(ethylsulfonamido)phenyl)-2′-(N-(t-butoxycarbonylamino))acetamido]-3-chloro-3-(1-carba-1-dethiacephem)-4-carboxylate (600 mg, 0.87 mmol) was dissolved in ethyl acetate (25 ml) and methanol (5 ml). Five percent palladium on carbon (300 mg) was suspended in ethanol (10 ml), methanol (2 ml), and ethyl acetate (2 ml). The mixture of palladium on carbon was subjected to a hydrogen pressure of 40 psi for 10 minutes. To this slurry was added the ethyl acetate/methanol solution of the pNB ester and the suspension was subjected to 40 psi of hydrogen pressure for 2 hours. The reaction mixture was filtered and filtrate was concentrated in vacuo to yield 520 mg of a foam of 7β-[2'-(R,S)-2'-(m-(ethylsulfonamido)phenyl)-2'-(N-(t-butoxycarbonylamino))acetamido]-3-chloro-3-(1-carba-1-dethiacephem)-4-carboxylic acid.

Reference Example 3

7β-[2'-(R,S)-2'-(m-(Ethylsulfonamido)phenyl)-2'-Aminoacetamido]-3-Chloro-3-(1-Carba-1-Dethiacephem)-4-Carboxylic Acid Trifluoroacetate Salt

7β-[2'-(R,S)-2'-(m-(Ethylsulfonamido)phenyl)-2'-(N-(t-butoxycarbonylamino))acetamido]-3-chloro-3-(1-carba-1-dethiacephem)-4-carboxylic acid (520 mg) was combined at room temperature with trifluoroacetic acid (15 ml). The solution was allowed to stand for 10 minutes, then evaporated in vacuo. Diethyl ether (50 ml) was added and the solution was stirred and sonicated to obtain a precipitate. The precipitate was collected by filtration and dried under vacuum at 40 ° C for 10 minutes to yield 350 mg, 64.2% of 7β-[2'-(R,S)-2'-(m-(ethylsulfonamido)phenyl)-2'-aminoacetamido]-3-chloro-3-(1-carba-1-dethiacephem)-4-carboxylic acid trifluoroacetate salt.

Example 4

7β-[2′-(R)-2′-(m-(Ethylsulfonamido)phenyl)-2′-Aminoacetamido)-3-Chloro-3-(1-Carba-1-Dethiacephem)-4-Carboxylic Acid, Zwitterionic Salt

7β-[2′-(R,S)-2′-(m-(Ethylsulfonamido)phenyl)-2′-aminoacetamido]-3-chloro-3-(1-carba-1-dethiacephem)-4-carboxylic acid trifluoroacetate salt (165 mg) was dissolved in water (1.5 ml) and few drops of acetonitrile. The solution was subjected to analytical-scale HPLC on $C_{18}$ reverse phase column eluted with a gradient of water versus acetonitrile. Several separations on this system yielded 10 mg of 7β-[2′-(R)-2′-(m-(ethylsulfonamido)phenyl)-2′-aminoacetamido]-3-chloro-3-(1-carba-1-dethiacephem)-4-carboxylic acid, zwitterionic salt: n.m.r. (500 MHz, DMSO-$d_6$): δ 1.18 (t, 3), 1.49/1.44 (m, 2), 2.46/2.27 (m, 2), 3.10 (m, 3), 3.69 (m, 1), 4.60 (s, 1), 5.22 (broad d, 1, J = 4.55 Hz), 7.12, 7.16, 7.28, 7.30 (m, 5).

Example 5

7β-[2′-(R)-2′-(m-(n-Propylsulfonamido)phenyl)-2′-Aminoacetamido]-3-Chloro-3-(1-Carba-1-Dethiacephem)-4-Carboxylic Acid, Zwitterionic Salt.

In a manner similar to the above Examples and Preparations, approximately 100 mg of the title product was obtained: n.m.r. (300 MHz, DMSO-$d_6$) δ: 0.9 (t), 1.4 (m), 1.65 (m), 2.3 (d), 3.0 (t), 3.7 (m), 4.6 (s), 5.2 (s), 7.2 (m), 9.1 (s); f.a.b.m.s.: ($M+1^+$) = 471.

Example 6

7β-[2′-(R)-2′-(m-(iso-Propylsulfonamido)phenyl)-2′-Aminoacetamido]-3-Chloro-3-(1-Carba-1-Dethiacephem)-4-Carboxylic Acid, Zwitterionic Salt.

In a manner similar to the above Examples and Preparations, approximately 1.58 g, 66% yield of the m-(iso-propylsulfonamido) title product was obtained: n.m.r. (300 MHz, DMSO-$d_6$) δ: 1.2 (m), 1.4 (m), 2.3 (d), 3.8 (m), 4.7 (s), 5.3 (s), 7.2 (m), 9.2 (s); f.a.b.m.s.: ($M+1^+$) = 471; u.v. (EtOH): $\lambda_{max}$ = 264 nm; $\epsilon_{max}$ = 10100.

**Claims**

**Claims for the following Contracting States : BE, CH, DE, FR, GB, IT, LI, NL, SE**

**1.** A compound of the Formula I

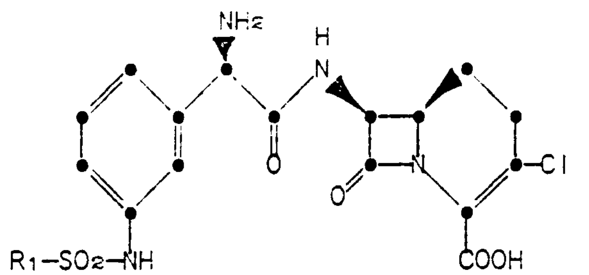

I

wherein $R_1$ is $C_1$ to $C_4$ alkyl,
or a pharmaceutically-acceptable salt thereof.

**2.** A compound of Formula I as claimed in claim, 1 wherein $R_1$ is methyl or ethyl.

**3.** 7β-[2′-(R)-2′-(m-(Methylsulfonamido)phenyl)-2′-aminoacetamido]-3-chloro-3-(1-carba-1-dethiacephem)-4-carboxylic acid.

**4.** 7β-[2′-(R)-2′-(m-(Ethylsulfonamido)phenyl)-2′-aminoacetamido]-3-chloro-3-(1-carba-1-dethiacephem)-4-carboxylic acid.

**5.** A pharmaceutical composition which comprises a compound as claimed in any one of the claims 1 to 4, or a pharmaceutically-acceptable salt thereof, associated with a pharmaceutically-acceptable carrier therefor.

**6.** A compound of Formula I, as claimed in any one of claims 1 to 4, or a pharmaceutically-acceptable salt thereof, for use in the chemotherapy of bacterial infections.

**7.** A process for preparing a compound of the Formula I as claimed in any one of claims 1 to 4 or a pharmaceutically-acceptable salt thereof, which comprises:
(A) the removal of the amino and/or carboxy protecting group from a compound of the formula II:

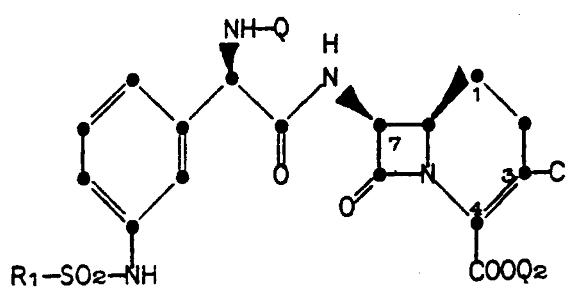

II

wherein Q is hydrogen or an amino-protecting group, and $Q_2$ is hydrogen or a carboxy-protecting group, provided that Q and $Q_2$ are not both hydrogen; or
(B) acylation of a compound of the formula

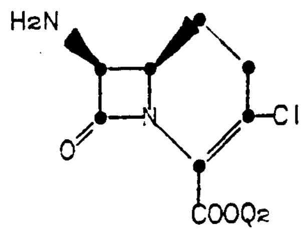

wherein $Q_2$ is as defined above followed, in the case where $Q_2$ is not hydrogen, by removal of the carboxy protecting group and any amino-protecting group present; and

(C) resolution of a racemic mixture of a compound of formula II where Q and $Q_2$ are both hydrogen.

**8.** A compound of formula II as defined in claim 7 provided that Q and $Q_2$ cannot both be hydrogen.

**Claims for the following Contracting State : AT**

**1.** A process for preparing a compound of the Formula I

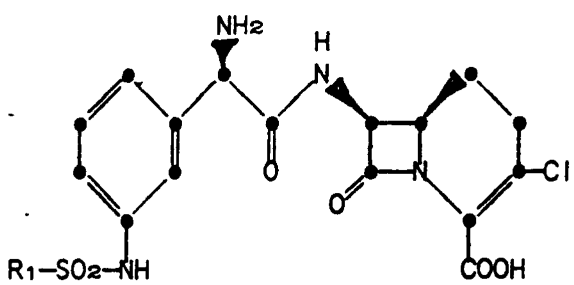

I

wherein $R_1$ is $C_1$ to $C_4$ alkyl,

or a pharmaceutically-acceptable salt thereof, which comprises:

(A) the removal of the amino and/or carboxy protecting group from a compound of the formula II:

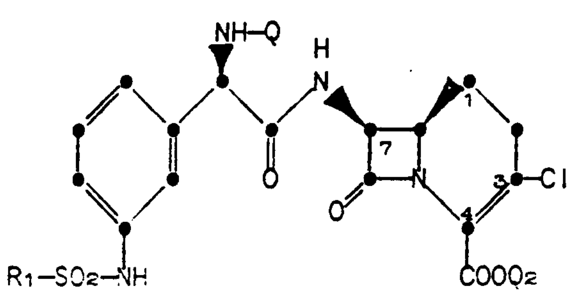

II

wherein Q is hydrogen or an amino-protecting group, and $Q_2$ is hydrogen or a carboxy-protecting group, provided that Q and $Q_2$ are not both hydrogen; or

(B) acylation of a compound of the formula

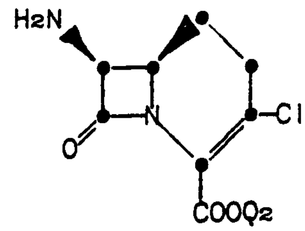

wherein $Q_2$ is as defined above followed, in the case where $Q_2$ is not hydrogen, by removal of the carboxy protecting group and any amino-protecting group present; and

(C) resolution of a racemic mixture of a compound of formula II where Q and $Q_2$ are both hydrogen.

**2.** A process according to claim 1 for preparing compound of Formula I as claimed in claim 1, wherein $R_1$ is methyl or ethyl.

**3.** A process according to claim 1 for preparing 7β-[2'(R)-2'-(m-(methylsulfonamido)phenyl)-2'-aminoacetamido]-3-chloro-3-(1-carba-1-dethiacephem)-4-carboxylic acid.

**4.** A process according to claim 1 for preparing 7β-[2'-(R)2'-(m-(ethylsulfonamido)phenyl)-2'-aminoacetamido]-3-chloro-3-(1-carba-1-dethiacephem)-4-carboxylic acid.

**Claims for the following Contracting State : ES**

**1.** A process for preparing a compound of the Formula I

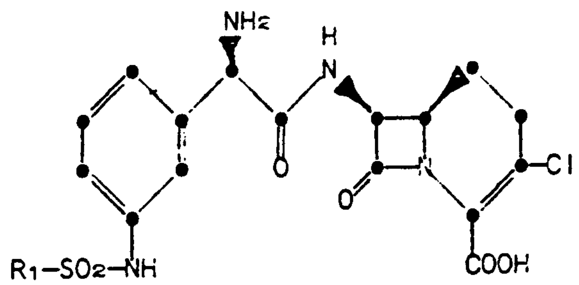

I

wherein $R_1$ is $C_1$ to $C_4$ alkyl,

or a pharmaceutically-acceptable salt thereof, which comprises:

(A) the removal of the amino and/or carboxy protecting group from a compound of the formula II:

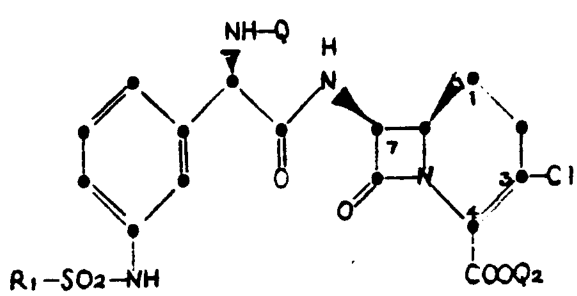

II

wherein Q is hydrogen or an amino-protecting group, and $Q_2$ is hydrogen or a carboxy-protecting group, provided that Q and $Q_2$ are not both hydrogen; or

(B) acylation of a compound of the formula

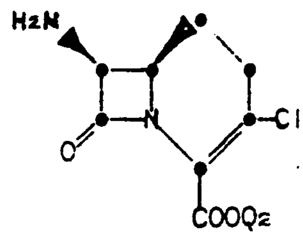

wherein $Q_2$ is as defined above followed, in the case where $Q_2$ is not hydrogen, by removal of the carboxy protecting group and any amino-protecting group present; and

(C) resolution of a racemic mixture of a compound of formula II where Q and $Q_2$ are both hydrogen.

**2.** A process according to claim 1 for preparing compound of Formula I as claimed in claim 1, wherein $R_1$ is methyl or ethyl.

**3.** A process according to claim 1 for preparing 7β-[2'(R)-2'-(m-(methylsulfonamido)phenyl)-2'-aminoacetamido]-3-chloro-3-(1-carba-1-dethiacephem)-4-carboxylic acid.

**4.** A process according to claim 1 for preparing 7β-[2'(R)-2'-(m-(ethylsulfonamido)phenyl)-2'-aminoacetamido]-3-chloro-3-(1-carba-1-dethiacephem)-4-carboxylic acid.

**5.** A process for preparing a pharmaceutical composition which comprises admixing a compound of formula (I) as defined in any one of claims 1 to 4, or a pharmaceutically-acceptable salt thereof, with a pharmaceutically-acceptable carrier therefor.

**Claims for the following Contracting State : GR**

**1.** A process for preparing a compound of the Formula I

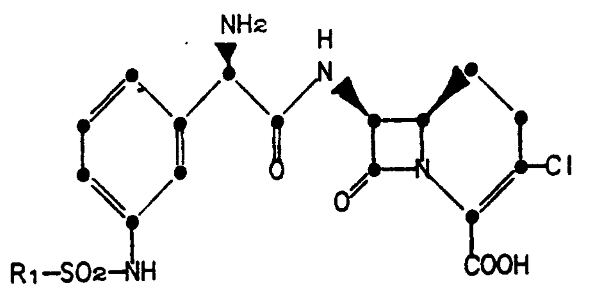

I

wherein $R_1$ is $C_1$ to $C_4$ alkyl,

or a pharmaceutically-acceptable salt thereof, which comprises:

(A) the removal of the amino and/or carboxy protecting group from a compound of the formula II:

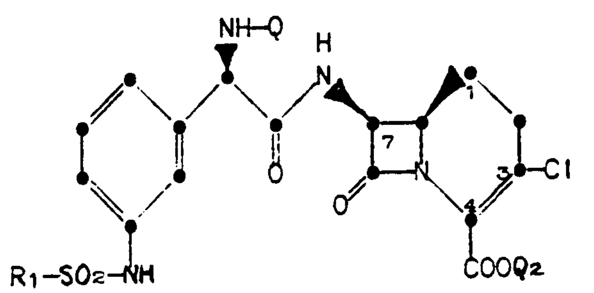

II

wherein Q is hydrogen or an amino-protecting group, and $Q_2$ is hydrogen or a carboxy-protecting group, provided that Q and $Q_2$ are not both hydrogen; or

(B) acylation of a compound of the formula

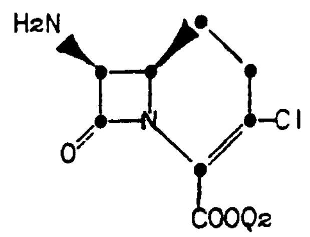

wherein $Q_2$ is as defined above followed, in the case where $Q_2$ is not hydrogen, by removal of the carboxy protecting group and any amino-protecting group present; and

(C) resolution of a racemic mixture of a compound of formula II where Q and $Q_2$ are both hydrogen.

**2.** A process according to claim 1 for preparing compound of Formula I as claimed in claim 1, wherein $R_1$ is methyl or ethyl.

**3.** A process according to claim 1 for preparing 7β-[2'-(R)-2'-(m-(methylsulfonamido)phenyl)-2'-aminoacetamido]-3-chloro-3-(1-carba-1-dethiacephem)-4-carboxylic acid.

**4.** A process according to claim 1 for preparing 7β-[2'(R)-2'-(m-(ethylsulfonamido)phenyl)-2'-aminoacetamido]-3-chloro-3-(1-carba-1-dethiacephem)-4-carboxylic acid.

**5.** A compound of formula II as defined in claim 1 provided that Q and $Q_2$ cannot both be hydrogen.

**6.** A process for preparing a pharmaceutical composition which comprises admixing a compound of formula (1) as defined in any one of claims 1 to 4, or a pharmaceutically-acceptable salt thereof, with a pharmaceutically-acceptable carrier therefor.

**Revendications**

**Revendications pour les Etats contractants suivants : BE, CH, DE, FR, GB, IT, LI, NL, SE**

**1.** Composé de formule I

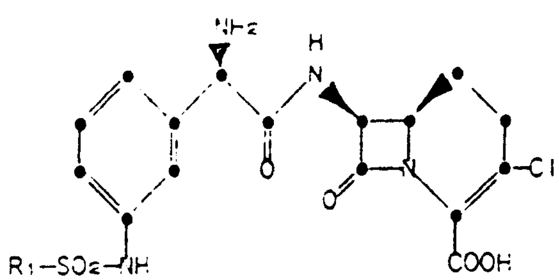

I

dans laquelle $R_1$ représente un groupe alkyle en $C_1$-$C_4$, ou un de ses sels pharmaceutiquement acceptable.

**2.** Composé de formule I selon la revendication 1, dans lequel $R_1$ représente un groupe méthyle ou un groupe éthyle.

**3.** Acide 7β-[2'(R)2'-(m-(méthylsulfonamido)-phényl)-2'-aminoacétamido]-3-chloro-3-(1-carba-1-déthiacéphem)-4-carboxylique.

4. Acide 7β-(2'-(R)-2'-(m-(éthylsulfonamido)-phényl)-2'-aminoacétamido]-3-chloro-3-(1-carba-1-déthiacéphem)-4-carboxylique.

5. Composition pharmaceutique qui comprend un composé selon l'une quelconque des revendications 1 à 4, ou un de ses sels pharmaceutiquement acceptables, en association avec un support pharmaceutiquement acceptable pour le composé.

6. Composé de formule I selon l'une quelconque des revendications 1 à 4 ou un de ses sels pharmaceutiquement acceptables, destiné à être utilisé dans la chimiothérapie d'infections bactériennes.

7. Procédé pour préparer un composé de formule I selon l'une quelconque des revendications 1 à 4 ou un de ses sels pharmaceutiquement acceptables, qui comprend :
(A) l'élimination du groupe protecteur du groupe amino et/ou du groupe carboxyle d'un composé de formule II :

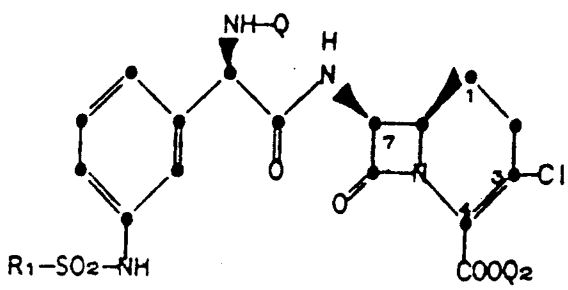

II

dans laquelle Q représente un atome d'hydrogène ou un groupe protecteur du groupe amino et $Q_2$ représente un atome d'hydrogène ou un groupe protecteur du groupe carboxyle à la condition que Q et $Q_2$ ne représentent pas, tous deux, un atome d'hydrogène;
ou
(B) l'acylation d'un composé de formule :

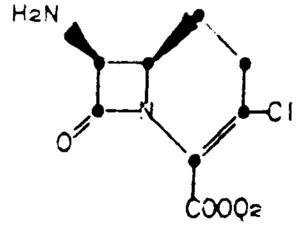

dans laquelle $Q_2$ est tel que défini ci-dessus, l'acylation étant suivie, dans le cas où $Q_2$ ne représente pas un atome d'hydrogène, par l'élimination du groupe protecteur du groupe carboxyle et de n'importe quel groupe présent protecteur du groupe amino; et
(C) le dédoublement d'un composé racémique de formule II dans laquelle Q et $Q_2$ représentent tous deux un atome d'hydrogène.

8. Composé de formule II, tel que défini dans la revendication 7, avec cette réserve que Q et $Q_2$ ne peuvent pas représenter, tous deux, un atome d'hydrogène.

**Revendication pour l'Etat contractant suivant : AT**

**1.** Procédé pour préparer un composé de formule I

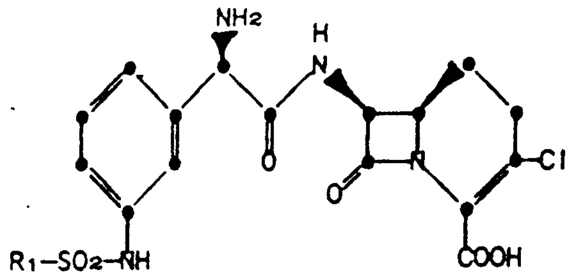

I

dans laquelle $R_1$ représente un groupe alkyle en $C_1$-$C_4$, ou un de ses sels pharmaceutiquement acceptable, qui comprend :

(A) l'élimination du groupe protecteur du groupe amino et/ou du groupe carboxyle d'un composé de formule II :

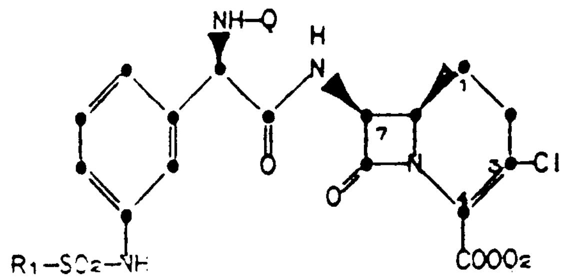

II

dans laquelle Q représente un atome d'hydrogène ou un groupe protecteur du groupe amino et $Q_2$ représente un atome d'hydrogène ou un groupe protecteur du groupe carboxyle à la condition que Q et $Q_2$ ne représentent pas, tous deux, un atome d'hydrogène;

ou

(B) l'acylation d'un composé de formule :

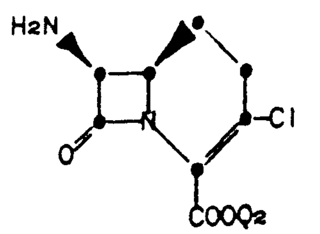

dans laquelle $Q_2$ est tel que défini ci-dessus, l'acylation étant suivie, dans le cas où $Q_2$ ne représente pas un atome d'hydrogène, par l'élimination du groupe protecteur du groupe carboxyle et de n'importe quel groupe présent protecteur du groupe amino; et

(C) le dédoublement d'un composé racémique de formule II dans laquelle Q et $Q_2$ représentent tous deux un atome d'hydrogène.

**2.** Procédé selon la revendication 1, pour préparer un composé de formule I selon la revendication 1, dans laquelle $R_1$ représente un groupe méthyle ou un groupe éthyle.

**3.** Procédé selon la revendication 1, pour préparer l'acide 7$\beta$-[2'-(R)-2'-(m-(méthylsulfonamido)-phényl)-2'-aminoacétamido]-3-chloro-3-(1-carba-1-déthiacéphem)-4-carboxylique.

4. Procédé selon la revendication 1, pour préparer l'acide 7β-[2'-(R)-2'-(m-(éthylsulfonamido)-phényl)-2'-aminoacétamido]-3-chloro-3-(1-carba-1-déthiacéphem)-4-carboxylique.

**Revendications pour l'Etat contractant suivant : ES**

1. Procédé pour préparer un composé de formule I

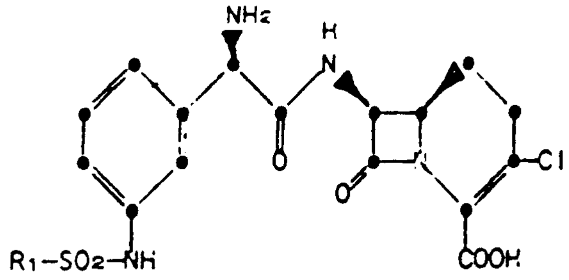

I

dans laquelle $R_1$ représente un groupe alkyle en $C_1$-$C_4$, ou un de ses sels pharmaceutiquement acceptable, qui comprend :

(A) l'élimination du groupe protecteur du groupe amino et/ou du groupe carboxyle d'un composé de formule II :

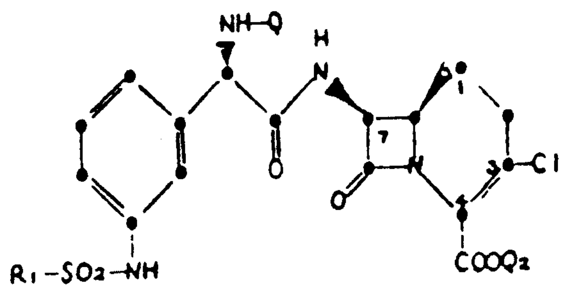

II

dans laquelle Q représente un atome d'hydrogène ou un groupe protecteur du groupe amino et $Q_2$ représente un atome d'hydrogène ou un groupe protecteur du groupe carboxyle à la condition que Q et $Q_2$ ne représentent pas, tous deux, un atome d'hydrogène;

ou

(B) l'acylation d'un composé de formule :

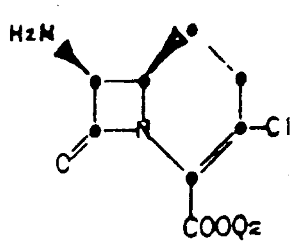

dans laquelle $Q_2$ est tel que défini ci-dessus, l'acylation étant suivie, dans le cas où $Q_2$ ne représente pas un atome d'hydrogène, par l'élimination du groupe protecteur du groupe carboxyle et de n'importe quel groupe présent protecteur du groupe amino; et

(C) le dédoublement d'un composé racémique de formule II dans laquelle Q et $Q_2$ représentent tous deux un atome d'hydrogène.

**2.** Procédé selon la revendication 1, pour préparer un composé de formule I selon la revendication 1, dans laquelle $R_1$ représente un groupe méthyle ou un groupe éthyle.

**3.** Procédé selon la revendication 1, pour préparer l'acide 7$\beta$-[2'-(R)-2'-(m-(méthylsulfonamido)-phényl)-2'-aminoacétamido]-3-chloro-3-(1-carba-1-déthiacéphem)-4-carboxylique.

**4.** Procédé selon la revendication 1, pour préparer l'acide 7$\beta$-[2'-(R)-2'-(m-(éthylsulfonamido)-phényl)-2'-aminoacétamido]-3-chloro-3-(1-carba-1-déthiacéphem)-4-carboxylique.

**5.** Procédé pour préparer une composition pharmaceutique qui consiste à mélanger un composé de formule I, tel que défini dans l'une quelconque des revendications 1 à 4, ou un de ses sels pharmaceutiquement acceptables, avec un support pharmaceutiquement acceptable pour le composé.

**Revendication pour l'Etat contractant suivant : GR**

**1.** Procédé pour préparer un composé de formule I

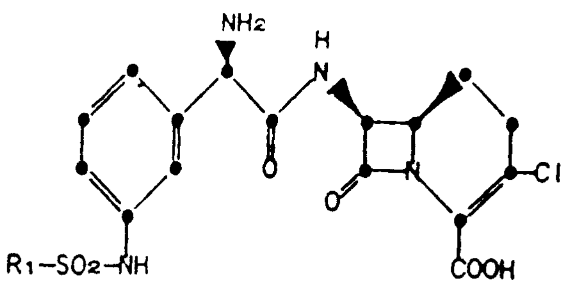

I

dans laquelle $R_1$ représente un groupe alkyle en $C_1$-$C_4$, ou un de ses sels pharmaceutiquement acceptable, qui comprend :

(A) l'élimination du groupe protecteur du groupe amino et/ou du groupe carboxyle d'un composé de formule II :

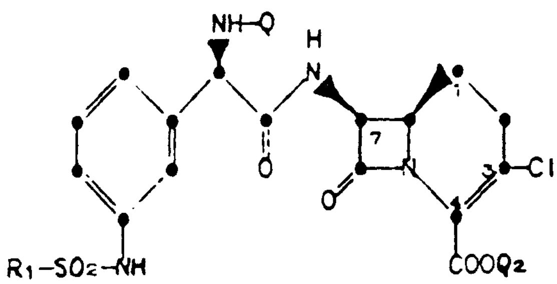

II

dans laquelle Q représente un atome d'hydrogène ou un groupe protecteur du groupe amino et $Q_2$ représente un atome d'hydrogène ou un groupe protecteur du groupe carboxyle à la condition que Q et $Q_2$ ne représentent pas, tous deux, un atome d'hydrogène;

ou

(B) l'acylation d'un composé de formule :

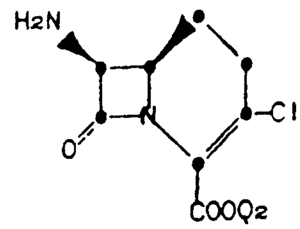

dans laquelle $Q_2$ est tel que défini ci-dessus, l'acylation étant suivie, dans le cas où $Q_2$ ne représente pas un atome d'hydrogène, par l'élimination du groupe protecteur du groupe carboxyle et de n'importe quel groupe présent protecteur du groupe amino; et
(C) le dédoublement d'un composé racémique de formule II dans laquelle Q et $Q_2$ représentent tous deux un atome d'hydrogène.

**2.** Procédé selon la revendication 1, pour préparer un composé de formule I selon la revendication 1, dans laquelle $R_1$ représente un groupe méthyle ou un groupe éthyle.

**3.** Procédé selon la revendication 1, pour préparer l'acide 7$\beta$-[2'-(R)-2'-(m-(méthylsulfonamido)-phényl)-2'-aminoacétamido]-3-chloro-3-(1-carba-1-déthiacéphem)-4-carboxylique.

**4.** Procédé selon la revendication 1, pour préparer l'acide 7$\beta$-(2'-(R)-2'-(m-(éthylsulfonamido)-phényl)-2'-aminoacétamido)-3-chloro-3-(1-carba-1-déthiacéphem)-4-carboxylique.

**5.** Composé de formule II, tel que défini dans la revendication 1, avec cette réserve que Q et $Q_2$ ne peuvent représenter tous deux un atome d'hydrogène.

**6.** Procédé pour préparer une composition pharmaceutique qui consiste à mélanger un composé de formule I, tel que défini dans l'une quelconque des revendications 1 à 4, ou un de ses sels pharmaceutiquement acceptables, avec un support pharmaceutiquement acceptable pour le composé.

**Patentansprüche**
**Patentansprüche für folgende Vertragsstaaten : BE, CH, DE, FR, GB, IT, LI, NL, SE**

**1.** Verbindung der Formel I

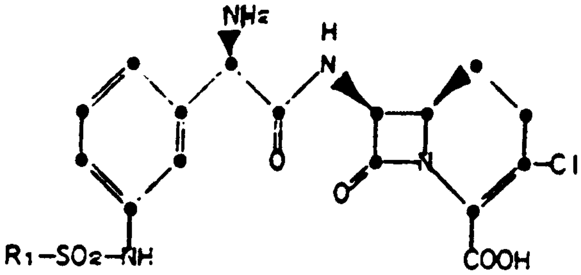

I

worin $R_1$ ein $C_1$-$C_4$-Alkylrest ist, oder ein pharmazeutisch annehmbares Salz davon.

**2.** Verbindung der Formel I nach Anspruch 1, worin $R_1$ ein Methyl- oder Ethylrest ist.

**3.** 7$\beta$-[2'(R)2'(m-(Methylsulfonamido)phenyl)-2'-aminoacetamido]-3-chlor-3-(1-carba-1-dethiacephem)-4-carbonsäure.

**4.** 7$\beta$[2'-(R)-2'-(m-(Ethylsulfonamido)phenyl)-2'-aminoacetamido]-3-chlor-3-(1-carba-1-dethiacephem)-4-carbonsäure.

5. Pharmazeutische Zusammensetzung umfassend eine Verbindung nach einem der Ansprüche 1 bis 4 oder ein pharmazeutisch annehmbares Salz davon Zusammen mit einem pharmazeutisch annehmbaren Träger.

6. Verbindung der Formel I nach einem der Ansprüche 1 bis 4 oder pharmazeutisch annehmbare Salze davon zur Verwendung in der Chemotherapie bakterieller Infektionen.

7. Verfahren zur Herstellung einer Verbindung der Formel I nach einem der Ansprüche 1 bis 4 oder eines pharmazeutisch annehmbaren Salzes davon, umfassend:
(A) die Entfernung der Amino- und/oder Carboxyschutzgruppe bei einer Verbindung der Formel II:

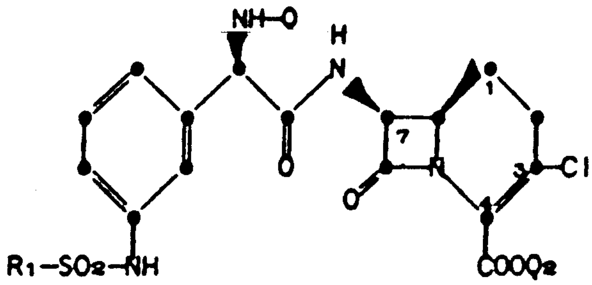

II

worin Q Wasserstoff oder eine Aminoschutzgruppe und $Q_2$ Wasserstoff oder eine Carboxyschutzgruppe ist, mit dem Vorbehalt, daß nicht beide Reste Q und $Q_2$ Wasserstoff sind, oder
(B) Acylierung einer Verbindung der Formel

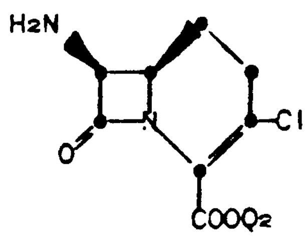

worin $Q_2$ wie oben definiert ist, und anschließend in dem Fall, wenn $Q_2$ nicht Wasserstoff ist, Entfernung der Carboxyschutzgruppe und jeder vorhandenen Aminoschutzgruppe, und
(c) Auftrennung einer razemischen Verbindung der Formel II, wenn Q und $Q_2$ beide Wasserstoff sind.

8. Verbindung der Formel II, wie in Anspruch 7 definiert, mit dem Vorbehalt, daß Q und $Q_2$ nicht beide Wasserstoff sein können.

**Patentansprüche für folgenden Vertragsstaat : AT**

1. Verfahren zur Herstellung einer Verbindung der Formel I

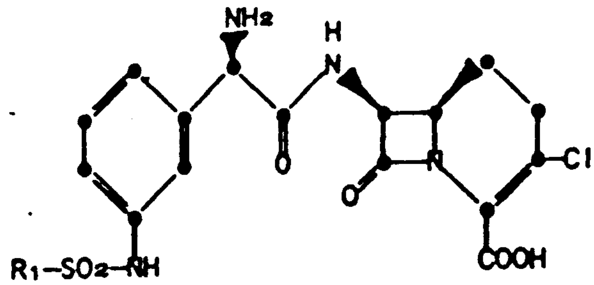

I

worin $R_1$ ein $C_1$-$C_4$-Alkylrest ist, oder eines pharmazeutisch annehmbaren Salzes davon, umfassend:

(A) die Entfernung der Amino- und/oder Carboxyschutzgruppe bei einer Verbindung der Formel II:

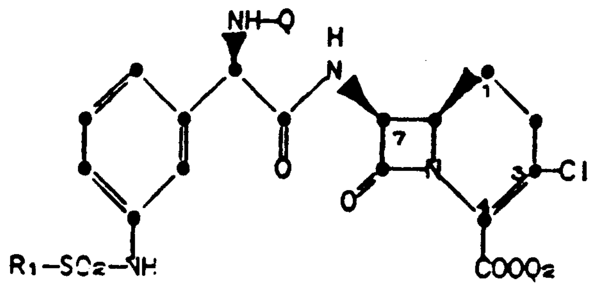

II

worin Q Wasserstoff oder eine Aminoschutzgruppe und $Q_2$ Wasserstoff oder eine Carboxyschutzgruppe ist, mit dem Vorbehalt, daß nicht beide Reste Q und $Q_2$ Wasserstoff sind, oder

(B) Acylierung einer Verbindung der Formel

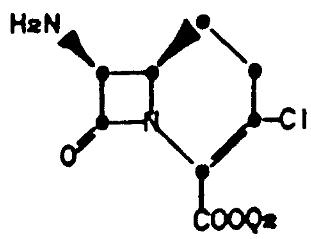

worin $Q_2$ wie oben definiert ist, und anschließend in dem Fall, wenn $Q_2$ nicht Wasserstoff ist, Entfernung der Carboxyschutzgruppe und jeder vorhandenen Aminoschutzgruppe, und

(C) Auftrennung einer razemischen Verbindung der Formel II, wenn Q und $Q_2$ beide Wasserstoff sind.

**2.** Verfahren nach Anspruch 1 zur Herstellung einer Verbindung der Formel I nach Anspruch 1, worin $R_1$ ein Methyl- oder Ethylrest ist.

**3.** Verfahren nach Anspruch 1 zur Herstellung von 7β-[2'-(R)-2'-(m-(Methylsulfonamido)phenyl)-2'-aminoacetamido]-3-chlor-3-(1-carba-1-dethiacephem)-4-carbonsäure.

**4.** Verfahren nach Anspruch 1 zur Herstellung von 7β-(2'-(R)-2'-(m-(Ethylsulfonamido)phenyl)-2'-aminoacetamido]-3-chlor-3-(1-carba-1-dethiacephem)-4-carbonsäure.

**Patentansprüche für folgenden Vertragsstaat : ES**

**1.** Verfahren zur Herstellung einer Verbindung der Formel I

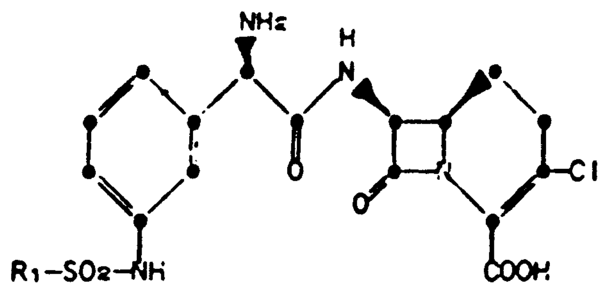

I

worin $R_1$ ein $C_1$-$C_4$-Alkylrest ist, oder eines pharmazeutisch annehmbaren Salzes davon, umfassend:

(A) die Entfernung der Amino- und/oder Carboxyschutzgruppe bei einer Verbindung der Formel II:

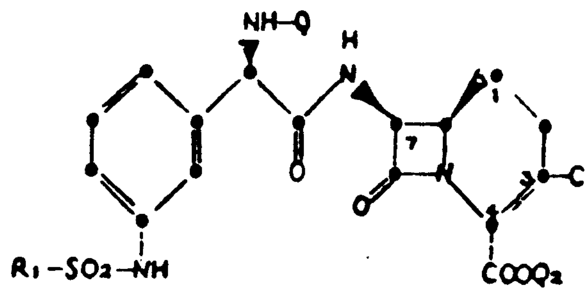

II

worin Q Wasserstoff oder eine Aminoschutzgruppe und $Q_2$ Wasserstoff oder eine Carboxyschutzgruppe ist, mit dem Vorbehalt, daß nicht beide Reste Q und $Q_2$ Wasserstoff sind, oder

(B) Acylierung einer Verbindung der Formel

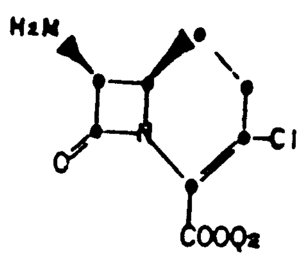

worin $Q_2$ wie oben definiert ist, und anschließend in dem Fall, wenn $Q_2$ nicht Wasserstoff ist, Entfernung der Carboxyschutzgruppe und jeder vorhandenen Aminoschutzgruppe, und

(C) Auftrennung einer razemischen Verbindung dem Formel II, wenn Q und $Q_2$ beide Wasserstoff sind.

**2.** Verfahren nach Anspruch 1 zur Herstellung einer Verbindung der Formel I nach Anspruch 1, worin $R_1$ ein Methyl- oder Ethylrest ist.

**3.** Verfahren nach Anspruch 1 zur Herstellung von 7$\beta$-[2'-(R)-2'-(m-(Methylsulfonamido)phenyl)-2'-aminoacetamido]-3-chlor-3-(1carba-1-dethiacephem)-4-carbonsäure.

**4.** Verfahren nach Anspruch 1 zur Herstellung von 7$\beta$-[2'-(R)-2'-(m-(Ethylsulfonamido)phenyl)-2'-aminoacetamido]-3-chlor-3-(1-carba-1-dethiacephem)-4-carbonsäure.

**5.** Verfahren zur Herstellung einer pharmazeutischen Zusammensetzung, umfassend, daß man eine Verbindung der Formel I, wie in einem der Ansprüche 1 bis 4 definiert, oder ein pharmazeutisch

annehmbares Salz davon mit einem pharmazeutisch annehmbaren Träger vermischt.

**Patentansprüche für folgenden Vertragsstaat : GR**

1. Verfahren zur Herstellung einer Verbindung der Formel I

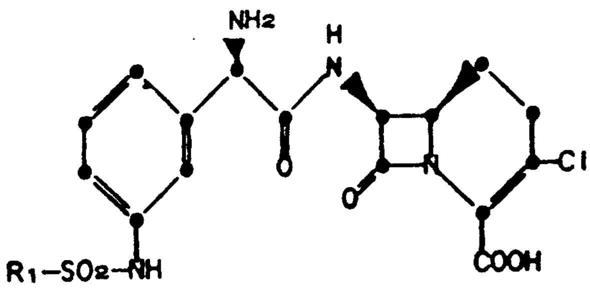

I

worin $R_1$ ein $C_1$-$C_4$-Alkylrest ist, oder eines pharmazeutisch annehmbaren Salzes davon, umfassend:
(A) die Entfernung der Amino- und/oder Carboxyschutzgruppe bei einer Verbindung der Formel II:

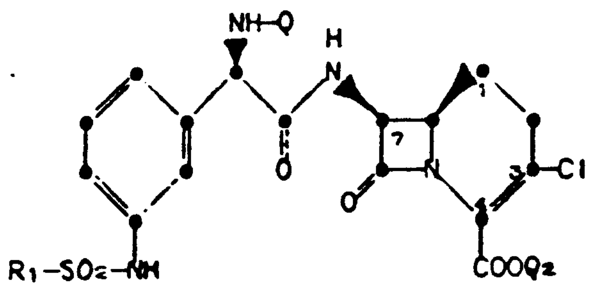

II

worin Q Wasserstoff oder eine Aminoschutzgruppe und $Q_2$ Wasserstoff oder eine Carboxyschutzgruppe ist, mit dem Vorbehalt, daß nicht beide Reste Q und $Q_2$ Wasserstoff sind, oder
(B) Acylierung einer Verbindung der Formel

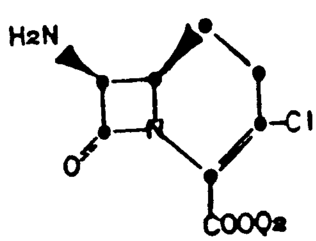

worin $Q_2$ wie oben definiert ist, und anschließend in dem Fall, wenn $Q_2$ nicht Wasserstoff ist, Entfernung der Carboxyschutzgruppe und jeder vorhandenen Aminoschutzgruppe, und
(C) Auftrennung einer razemischen Verbindung der Formel II, wenn Q und $Q_2$ beide Wasserstoff sind.

2. Verfahren nach Anspruch 1 zur Herstellung einer Verbindung der Formel I nach Anspruch 1, worin $R_1$ ein Methyl- oder Ethylrest ist.

3. Verfahren nach Anspruch 1 zur Herstellung von 7β-[2'-(R)-2'-(m-(Methylsulfonamido)phenyl)-2'-aminoacetamido]-3-chlor-3-(1-carba-1-dethiacephem)-4-carbonsäure.

**4.** Verfahren nach Anspruch 1 zur Herstellung von 7*β*-[2'-(R)-2'-(m-(Ethylsulfonamido)phenyl)-2'-aminoacetamido]-3-chlor-3-(1-carba-1-dethiacephem)-4-carbonsäure.

**5.** Verbindung der Formel II, wie in Anspruch 1 definiert, mit dem Vorbehalt, daß Q und $Q_2$ nicht beide Wasserstoff sein können.

**6.** Verfahren zur Herstellung einer pharmazeutischen Zusammensetzung, umfassend, daß man eine Verbindung der Formel I, wie in einem der Ansprüche 1 bis 4 definiert, oder ein pharmazeutisch annehmbares Salz davon mit einem pharmazeutisch annehmbaren Träger vermischt.